(19) Europäisches Patentamt
European Patent Office
Office européen des brevets

(11) **EP 2 912 956 A1**

(12) **EUROPEAN PATENT APPLICATION**
published in accordance with Art. 153(4) EPC

(43) Date of publication:
**02.09.2015 Bulletin 2015/36**

(21) Application number: **13848869.7**

(22) Date of filing: **21.10.2013**

(51) Int Cl.:
*A23L 1/304* (2006.01)   *C02F 1/28* (2006.01)
*C02F 1/44* (2006.01)

(86) International application number:
**PCT/KR2013/009388**

(87) International publication number:
**WO 2014/065550 (01.05.2014 Gazette 2014/18)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **24.10.2012 KR 20120118453**

(71) Applicant: **Aribio Inc.**
**Seoul 150-095 (KR)**

(72) Inventors:
• **CHOUNG, Jai Jun**
  **Seongnam-si**
  **Gyeonggi-do 463-743 (KR)**
• **SUNG, Soo Hyun**
  **Seoul 134-877 (KR)**
• **KU, Sae Kwang**
  **Daegu 706-920 (KR)**

(74) Representative: **Brevalex**
**95, rue d'Amsterdam**
**75378 Paris Cedex 8 (FR)**

(54) **COMPOSITION FOR PREVENTING, ALLEVIATING OR TREATING CONSTIPATION, CONTAINING HIGH HARDNESS MINERAL WATER PREPARED FROM DEEP OCEAN WATER OR DESALINATED GROUNDWATER**

(57) The present invention relates to a functional composition for preventing, alleviating or treating constipation, containing high hardness mineral water prepared from deep ocean water or desalinated groundwater, and a method therefor. The present invention contains high hardness mineral water prepared from deep ocean water or desalinated groundwater as an active ingredient so as to be safe to the human body, and thus can be useful for treating constipation of a cancer patient and constipation of a regular person.

**Fig. 2**

EP 2 912 956 A1

**Description**

Technical Field

The present invention was made with the support of the Ministry of Education, Science, and Technology of Republic of Korea, under Project No. 2011-0030124, which was conducted with the research project entitled "Medical Research Center (MRC) of Leading Research Center Support Project" within the project named "Global Research Center for Control Science" by Daegu Haany University under the management of the National Research Foundation of Korea, from September 1, 2011 to August 31, 2012.

This application claims priority to and the benefit of Korean Patent Application No. 10-2012-0118453 filed in the Korean Intellectual Property Office on October 24, 2012, the disclosures of which are incorporated herein by reference.

The present invention relates to a composition and method for preventing, alleviating, or treating constipation, the composition containing high hardness mineral water prepared from deep ocean water or salty groundwater.

Background Art

Recently, an increasing number of people have complained of constipation, due to various factors including stress caused by industrialization, lack of dietary fiber resulting from an increase in the intake of meat and junk food caused by a westernized diet, and the like. From medical standpoint, constipation is when the frequency of bowel movements is less than three times a week or the amount of feces is 30 g or less. In a normal state, food is excreted as feces within 24 hours after ingestion. However, people with constipation have a bowel movement every few days, or have a small amount of feces even though they defecate, and resultantly, the intestinal residence time of ingested food is longer.

The constipation is assigned to various causes, such as decreased bowel peristalsis due to skipping breakfast, lack of dietary fiber due to a westernized diet, deterioration in basic physical strength and abdominal muscle strength due to insufficient exercise, going on a diet, and drug addiction due to drug overdose. Constipation, which is said to lead to all kinds of diseases from ancient times, accompanies loss of appetite, abdominal distension at all times, accelerated skin aging caused by absorption of the toxins of feces, which cannot be excreted, into the blood through the intestines, headaches, acne, skin rashes, or the like. Further, severe constipation causes piles, such as anal fissure and hemorrhoidal protrusion during bowel movements, and even leads to colon cancer due to toxins contained in the feces. Further, if constipation continues, cholesterol or fat, which needs to be excreted from the body, remains in the body, causing atherosclerosis or cholelithiasis, and further hypertension and cardiac hypertrophy, leading to heart disease. Besides, constipation causes various severe secondary diseases including stroke, immune deficiency, visual impairment, mental illness (depression), and the like, and thus is recognized as a major disease requiring aggressive prevention and treatment.

Currently, various medical products and functional foods for constipation treatment and alleviation are being marketed, and studies on the same are being conducted. For example, Korean Patent Registration No. 10-07206950 discloses a pharmaceutical composition containing, as essential ingredients, a mixed extract of Rehmanniae Radix Preparat, Ligustici Rhizoma, and Angelicae Gigantis Radix, and a Plantaginis Semen powder for treating and preventing constipation; and Korean Patent Application No. 10-2008-0012457 discloses a composition containing Coicis Radix, Atractylodis Rhizoma, Aloe, Rhei Rhizoma, honey, and propolis for treating constipation or abdominal obesity. In addition, Korean Patent Registration No. 10-0886598 discloses a composition containing a 15-keto-prostaglandin compound as an active ingredient for treating drug-induced constipation.

Meanwhile, deep ocean water refers to ocean water found and collected from a site 200 m below the surface, where sunlight does not reach. The deep ocean water does not mix with the surface water, like water and oil, since the temperature difference between the deep ocean water and the surface water is at least 20°C. The deep ocean water flowing in the deep sea has very little temperature variance throughout the year, thereby having low-temperature stability. In addition, since sunlight cannot touch the deep ocean water, photosynthesis does not happen in the deep ocean water. Therefore, the deep ocean water contains large quantities of inorganic nutrients, and especially, contains essential trace elements and various minerals, such as magnesium and calcium, in balance. In addition, it is known that since the deep ocean water is not polluted by artificial materials, there are less organic materials or pathogenic bacteria to be decomposed in deep ocean water, and thus the deep ocean water has fewer bacteria and retains high anti-microbial properties and deodorizing properties. Various studies on the use of deep ocean water, from which sodium chloride is removed, for beverages or foods, cosmetics, and the like have been conducted.

High-hardness mineral water is rich in minerals, but has a degraded eating texture (refreshing feeling). The reason for this is that a mineral solution added to the mineral water contains large quantities of $Cl^-$ and $SO_4^{2-}$, which degrade the eating texture, as well as useful minerals, such as calcium and magnesium. Moreover, the thus prepared mineral water may not meet the standard of drinking water with respect to the contents of sulfate and chloride ions.

These drawbacks are the main causes to restrict the manufacture of high-hardness mineral water having high contents of minerals and an excellent eating texture.

Throughout the entire specification, many papers and patent documents are referenced and their citations are represented. The disclosures of cited papers and patent documents are entirely incorporated by reference into the present specification, and the level of the technical field within which the present invention falls and details of the present invention are explained more clearly.

Detailed Description of the Invention

Technical Problem

The present inventors have endeavored to develop a material which is stable and has excellent effects of preventing, alleviating, and treating constipation. As a result, the present inventors confirmed that constipation symptoms can be significantly alleviated or treated by merely ingesting high-hardness mineral water prepared from deep ocean water or salty underground water.

Therefore, the present invention has been made in view of the above-mentioned problems, and an aspect of the present invention is to provide a composition containing, as an active ingredient, high-hardness mineral water prepared from deep ocean water or salty underground water for preventing or alleviating constipation.

Another aspect of the present invention is to provide a pharmaceutical composition for preventing or treating constipation, the pharmaceutical composition containing: a pharmaceutically effective amount of high-hardness mineral water prepared from deep ocean water or salty underground water; and a pharmaceutically acceptable carrier.

Still another aspect of the present invention is to provide a method for preventing, alleviating, or treating constipation.

Other purposes and advantages of the present disclosure will be clarified by the following detailed description of the invention, claims, and drawings.

Technical Solution

In accordance with an aspect of the present invention, there is provided a composition for preventing or alleviating constipation, containing, as an active ingredient, high-hardness mineral water prepared from deep ocean water or salty underground water, wherein the high-hardness mineral water has the following properties:

    (a) has a hardness value of 40-3000; and
    (b) contains 65-700 mg/l of magnesium, 20-280 mg/l of calcium, and 15-300 mg/l of potassium.

The present inventors have endeavored to develop a material which is stable and has excellent effects of preventing, alleviating, and treating constipation. As a result, the present inventors confirmed that constipation symptoms can be significantly alleviated or treated by merely ingesting high-hardness mineral water prepared from deep ocean water or salty underground water.

As used herein, the term "prevention" refers to all actions that inhibit or retard the progress of constipation by ingesting the composition of the present invention. The term "alleviation" refers to all actions that alleviate or beneficially change the symptoms of constipation by ingesting the composition of the present invention.

As used herein the term "constipation" refers to a condition in which a smooth bowel movement is not possible due to decreased bowel peristalsis, and preferably, refers to a condition of straining during a bowel movement, excessive thickening of feces, incomplete evacuation (uncomfortable feeling after defecation) or anorectal obstructive feeling, less than three bowel movements per week, or the like. Besides, the constipation of the present invention also encompasses an individual recognition of constipation symptoms.

As used herein, the term "deep ocean water" refers to water that is not less than 200 m deep, where sunlight does not reach. The term "salty underground water" refers to underground water in the bedrock aquifer containing more than a predetermined amount of dissolved solid, such as salts, and means raw water which is to be drunk in a natural state in which the water quality can be continuously maintained.

In the present invention, any salty underground water may be used without limitation, but it is preferable to use underground water in the bedrock aquifer, which has a total dissolved solid of 2000 mg/l, including a salt dissolved in the water.

As used herein, the term "hardness" refers to water strength which is generated by divalent metal cations, such as $Ca^{2+}$ and $Mg^{2+}$, dissolved in water and expressed as a value in terms of $CaCO_3$. Preferably, the hardness of the present invention is calculated by equation 6 below.

[Equation 6]

Water hardness ($CaCO_3$ mg/ℓ) = 2.5 × [$Ca^{2+}$ concentration (mg/ℓ)] + 4.1 ×

[$Mg^{2+}$ concentration (mg/ℓ)]

According to a preferable embodiment of the present invention, the high-hardness mineral water of the present invention may have a hardness (mg/l as $CaCO_3$) of 400-3000, preferably 410-2950, more preferably 420-2900, still more preferably 430-2850, still more preferably 440-2800, still more preferably 450-2750, still more preferably 460-2700, still more preferably 470-2650, still more preferably 480-2600, still more preferably 490-2550, still more preferably 500-2500, still more preferably 500-2400, still more preferably 500-2300, still more preferably 500-2200, still more preferably 500-2100, still more preferably 500-2000, still more preferably 500-1900, still more preferably 500-1800, still more preferably 500-1700, still more preferably 500-1600, still more preferably 500-1500, still more preferably 510-1500, still more preferably 520-1500, still more preferably 530-1500, still more preferably 540-1500, still more preferably 550-1500, still more preferably 560-1500, still more preferably 570-1500, still more preferably 580-1500, still more preferably 590-1500, still more preferably 600-1500.

According to a preferable embodiment of the present invention, the mineral water having a hardness of 100 of the present invention contains 15-30 mg/l of magnesium, 4.5-10 mg/l of calcium, and 4-12 mg/l of potassium; the mineral water having a hardness of 600 contains 101-140 mg/l of magnesium, 32-49 mg/l of calcium, and 29-54 mg/l of potassium; the mineral water having a hardness of 1500 contains 250-360 mg/l of magnesium, 75-125 mg/l of calcium, and 70-145 mg/l of potassium; and the mineral water having a hardness of 2000 contains 320-480 mg/l of magnesium, 95-170 mg/l of calcium, and 90-200 mg/l of potassium.

Preferably, the mineral water of the present invention has a hardness value of 500-2000, and contains 80-480 mg/l of magnesium, 25-170 mg/l of calcium, and 20-200 mg/l of potassium, and more preferably, a hardness value of 600-1500, and contains 101-360 mg/l of magnesium, 32-125 mg/l of calcium, and 29-145 mg/l of potassium.

According to a preferable embodiment of the present invention, the high-hardness mineral water of the present invention exhibits at least one activity selected from the group consisting of: (a) an increase in the weight of fecal pellets; (b) an increase in the water content of fecal pellets; (c) a decrease in the number of remnant fecal pellets in the colon; (d) an increase in the intestinal fecal pellet transit ratio; and (e) increases in the fecal pellet surface mucous thickness, colon mucosa thickness, and mucous-producing cell number per unit area in colon mucosa. As validated in the examples below, the mineral water of the present invention, at the time of administration, exhibits effects of: increasing the number of fecal pellets and the water content of the fecal pellets (table 5); decreasing the number and mean thickness of remnant fecal pellets in the colon (table 6); increasing the intestinal fecal pellet transit ratio (table 7); and increasing the fecal pellet surface mucous thickness, colon mucosa thickness, and mucous-producing cell number per unit area in colon mucosa.

The composition of the present invention can be applied to a subject in need of prevention or alleviation of constipation, and preferably, the term "subject" refers to all animals, including humans, that are constipated or may get constipated.

According to a preferable embodiment of the present invention, the composition of the present invention is used to prevent or alleviate the constipation for a cancer patient, and the cancer patient is preferably a cancer patient receiving chemotherapy. Anticancer drug *per se* may cause constipation, and taking antiemetic drug causes constipation as a side effect, and thus the food composition of the present invention can be used to alleviate constipation of a cancer patient who is suffering from constipation due to administration of anticancer drug. The anticancer drug encompasses all kinds of anticancer drugs, which are used to prevent, alleviate, or treat cancer, without limitation, and an example thereof may be cisplatin.

The mineral water of the present invention is characterized by having high hardness and containing large quantities of magnesium, calcium, and potassium ions. Such ions allow the absorption of moisture to drop viscosity of the blood, thereby helping blood circulation, contributing to the alleviation of the symptoms of constipation. However, the preparation of the mineral water containing such ions in large quantities has the following problems. As a first problem, in cases where a mineral concentrate is added in large quantities to desalted water in order to increase the mineral content in drinking water, the drinking water contains large quantities of sulfate and chlorine ions, which degrade the eating texture, as well as cations, such as magnesium ions or potassium ions. As a second problem, in cases where calcium salts separated from concentrate water are added to desalted water in order to supply calcium, elements of salt and calcium carbonate contained in the calcium salts are added to degrade the eating texture of the drinking water. Moreover, since the calcium salts have low solubility, the substantial supply efficiency of calcium is low. Therefore, although high-hardness mineral water is needed for effective prevention and alleviation of constipation, the current manufacturing of high-hardness mineral water is not easy due to the above problems. However, the present invention provides high-hardness mineral water having an excellent eating texture as drinking water and containing large quantities of magnesium ions and the like through the following procedure, thereby suggesting the possibility of high-hardness mineral water as prophylactic and therapeutic agents for constipation.

According to a preferable embodiment of the present invention, the high-hardness mineral water of the present invention is prepared to have a hardness of 400-3000 by mixing desalted water, which is obtained by performing a desalting treatment on deep ocean water or salty underground water, with (i) a mineral concentrate obtained by separating calcium salt crystals and salt from concentrated water obtained by performing a desalting treatment on deep ocean water or salty underground water; and (ii) calcium salts obtained by removing, from the calcium salt crystals separated from the concentrated water, salt put on the calcium salt crystals and calcium carbonate.

Respective steps will be described below.

The desalting treatment for separating desalted water and concentrated water from deep ocean water or salty underground water may be conducted by employing any technique known in the art. Techniques for the desalting treatment may be an evaporation method, a seawater freezing method, a reverse osmosis method, an ion exchange resin method, an electrodialysis method, and the like. Preferably, as for the desalting treatment, raw water is allowed to pass through a reverse osmotic membrane to be separated into concentrated water containing ion components and desalted water from which ion components are removed. The raw water may be used after having gone through a pretreatment process, such as filtration, for removing floating materials. The pretreatment process is conducted to remove impurities that may cause a membrane blockage at the time of reverse osmosis filtration, and typically, microfiltration or ultrafiltration may be conducted.

In the present invention, the desalted water may be obtained from the raw water by a desalting treatment with two or more steps. For example, the raw water is passed through a first reverse osmosis membrane to obtain first concentrated water and first desalted water, and the first desalted water is passed through a second reverse osmosis membrane to obtain second concentrated water and second desalted water. After that, calcium salts and a mineral concentrate, which are separated from the first concentrated water, are added to the second desalted water to prepare high-hardness mineral water.

The concentrated water contains calcium salts, such as calcium sulfate and calcium chloride, and sodium chloride, and minerals in large quantities. In the present invention, the calcium salts and salt are separated from the concentrated water separated from the raw water, stage by stage.

In the present invention, in order to extract calcium salt crystals and salt from the concentrated water, the concentrated water is heated and concentrated such that the concentrated water has an appropriate specific gravity value (a ratio of concentrated water density over water density under the same temperature and pressure), thereby separating the extracted calcium salt crystals and salt.

According to a preferable embodiment of the present invention, the concentrated water is heated and concentrated such that the specific gravity (a ratio of concentrated water density over water density under the same temperature and pressure) is 1.11 or more, thereby separating educed calcium salt crystals. The preferable specific gravity of the concentrated water to separate calcium salts is, but not limited to, 1.11-1.23. The separation of calcium salts may be conducted using a mesh net, and the separation may be conducted using preferably a 300- to 350-mesh net, and more preferably a 300-mesh net.

According to a preferable embodiment of the present invention, the concentrated water is heated and concentrated to have a specific gravity of 1.8, thereby first separating extracted calcium salts, and the filtrate is heated and concentrated to have a specific gravity of 1.19-1.23, thereby removing remnant calcium salts.

According to another preferable embodiment of the present invention, the concentrated water is heated such that it has a specific gravity of 1.23, and then is allowed to stand, thereby extracting calcium salts deposited on the bottom.

According to a preferable embodiment of the present invention, the separation of salt may be conducted, such that the concentrated water is heated and concentrated to have a specific gravity of 1.24, thereby separating the extracted salt. The preferable specific gravity of the concentrated water to separate salt is, but not limited to, 1.24-1.32. Since a mineral concentrate (liquid) and salt (solid) are present together in the heated concentrated water, the salt may be separated by centrifugation or by using a dehydrator. The separated salt may be processed into mineral salt by going through an additional purification process.

In the present invention, the heating of the concentrate water may be slowly conducted simultaneously with stirring.

In the present invention, the concentrated water, from which calcium salts and salt have been removed, may be further heated and concentrated in order to concentrate mineral components.

According to a preferable embodiment of the present invention, anions and impurities are removed from the mineral concentrate, from which calcium salts and elements of salt have been removed and which is to be mixed with desalted water, by a method for improving quality of the mineral concentrate, the method comprising: (a) filtering the mineral concentrate through a filter having a physical adsorbent; (b) re-filtering the filtered mineral concentrate through a filter having a physical adsorbent; and (c) filtering the mineral concentrate, which has been filtered in step (b), through a hollow fiber membrane filter having a plurality of pores. The removal of the impurities is absolutely necessary for the preparation of drinking water, and the removal of anions is required to improve the refreshing feeling of the mineral water. The present procedure is characterized by filtering the mineral concentrate through a filter having a physical adsorbent and then re-filtering the filtered mineral concentrate through a filter having a physical adsorbent. For the filters used for the filtering and re-filtering, the same filter may be reused, or separate filters may be used. In addition, the filter for the re-filtering may be different from the filter used for first filtering with respect to the filter length, constituents, kind of adsorbent, and/or size of micropores.

In the present invention, when the mineral concentrate passes through the filter having a physical adsorbent, anions (especially, chlorine ions and sulfate ions) and impurities (especially, silt) in the mineral concentrate are removed by being adsorbed on the plurality of micropores of the adsorbent, and the removing efficiency of anions and impurities is maximized through re-filtering using a filter having a physical adsorbent (first filtering). After that, the first-filtered mineral concentrate is second filtered through a hollow fiber membrane filter, thereby improving the removing efficiency of anions and impurities.

In the present invention, any filter having a physical adsorbent capable of adsorbing impurities and anions present in the solution may be used without limitation. Preferably, the physical adsorbent is activated carbon, diatomite, zeolite, silica gel, starch, bentonite, or alumina, and more preferably activated carbon.

In the present invention, an appropriate activated carbon may be selectively used depending on the amount of mineral concentrate. For example, preferably, an 8- to 12-inch activated carbon filter is used for treating 100 l of the mineral concentrate, and an 18- to 22-inch activated carbon filter is used for treating 200 l of the mineral concentrate. More preferably, a 10-inch activated carbon filter is used for treating 100 $\ell$ of the mineral concentrate, and a 20-inch activated carbon filter is used for treating 200 $\ell$ of the mineral concentrate.

In the present invention, the concentrate water which has been first filtered through the filter having a physical adsorbent is second filtered through a hollow fiber membrane filter, thereby further filtering out anions and impurities containing microorganisms and silt, and allowing the mineral components in the mineral concentrate to pass through the hollow fiber membrane filter. As the hollow fiber membrane filter, any micro-filter having a plurality of pores may be used without limitation, and the pores have a diameter of 0.01-0.5 $\mu$m, preferably 0.05-0.5 $\mu$m, and more preferably 0.1-0.5 $\mu$m.

Preferably, the hollow fiber membrane filter is a sterilizing filter.

According to a preferable embodiment of the present invention, silt and anions in the mineral concentrate are removed by the filtering, and preferably, silt, chlorine ions, and sulfate ions are removed. As validated in the examples below, the yellowish mineral concentrate was filtered through the activated carbon filter, the activated carbon filter, and the sterilizing filter, and thus is changed into colorless, which indicates that impurities including silt and the like were removed from the mineral concentrate. In addition, it was verified that the chlorine ions and sulfate ions were reduced by 6.52% and 2.08%, respectively, by the filtering (table 2).

In the present invention, in order to improve the removing efficiency of impurities and anions, a circulation procedure may be repeatedly performed: filtering the mineral concentrate through a filter having a physical adsorbent → refiltering the filtered concentrate through a filter having a physical adsorbent → rere-filtering the re-filtered concentrate. The number of repetitions is preferably one or more, more preferably 1-5 times, and still more preferably 1-4 times.

In the present invention, the mineral concentrate may be supplied into the filter by a supply unit at an appropriate rate or appropriate flow rate. The supply unit preferably employs a pump, and more preferably a metering pump that can deliver the mineral concentrate to the filter at a constant rate (or flow rate).

According to a preferable embodiment of the present invention, the removal of salt and calcium carbonate from calcium salt crystals separated from the concentrated water may be conducted by (i) putting the calcium salt crystals separated from the concentrated water into a container which contains hot water and is equipped with a 300- to 350-mesh net; and (ii) separating the calcium salt crystals which do not pass through the net. Here, the salt put on the calcium salt crystals is dissolved in the hot water, and the calcium carbonate passes through the mesh net and is deposited on the bottom in the container. The calcium salt crystals separated from the concentrated have been separated from the concentrated water containing large quantities of elements of salt, and thus necessarily contain the concentrated water. Therefore, in cases where the calcium salt crystals, without the removal of salt, are added to the desalted water, the salt component of the concentrated water degrades the eating texture of the mineral water. Moreover, the eating texture of the mineral water is degraded due to calcium carbonate. Thus, in the present invention, a purification procedure is performed to remove salt (concentrated water) put on the calcium salt crystals and calcium carbonate.

Preferably, in step (i), the container is slowly shaken after the calcium salt crystals are put into the container.

Preferably, a net having an equal or higher standard than the mesh net which has been used when the calcium salt crystals are extracted is used for the mesh net in step (i). More preferably, a 300-mesh (300 holes per inch) to 350-mesh net is used, and still more preferably, 300-mesh net is used.

The temperature of the solution in the container is set to a temperature at which the calcium salts can be extracted as crystals (the calcium salts can be present as crystals). When the temperature of the solution is lower than the above temperature, the calcium salts are dissolved in water, thereby lowering the filtering efficiency of carbon calcium by the mesh net. The temperature of the solution may be set to 60-100°C, preferably 65-100°C, and more preferably 70-100°C. The reason is that, since the calcium salts are extracted at 70-100°C, the same conditions are met.

The calcium salt crystals separated in step (ii) may be dried by natural drying, dry oven, a microwave oven, or the like, and then stored. Preferably, the calcium salt crystals are dried using a dry oven or a microwave oven. After that, the stored calcium salts may be added to desalted water to supply calcium.

As validated in the examples below, a larger quantity of calcium was detected in the distilled water where un-purified calcium salt crystals are dissolved than in the distilled water where the calcium salt crystals having gone through the purification procedure of the present invention are dissolved. This result indicates that, in cases where calcium salts separated from deep ocean water are purified by the method of the present invention, well-soluble pure calcium can be obtained. Thus, the calcium concentration of the mineral water can be efficiently improved.

In the present invention, high-hardness mineral water containing large quantities of magnesium, calcium, and potassium and having an excellent eating texture can be prepared by the foregoing method, and the prepared high-hardness mineral water can be useful for the prevention and/or treatment of constipation.

The composition of the present invention may be provided as a functional food composition.

The functional food composition of the present invention may be, but particularly limited to, any type of foods, such as drinking water, functional health food, dietary supplements, nutrients, pharma food, healthy food, nutraceutical, designer food, and food additives. Preferably, functional food composition of the present invention may be mineral water, drinking water, bread, chocolate, candies, snacks, cookies, pizza, ramen, other noodles, gum, dairy products including ice cream, various kinds of soups, teas, drinks, alcoholic beverages, and vitamin complexes, and may be added when manufacturing the normal foods known in the art.

The food composition of the present invention contains components that are normally added at the time of food manufacturing, in addition to high-hardness mineral water as an active ingredient, and contains, for example, proteins, carbohydrates, fats, nutrients, seasoning, and flavoring agents. Examples of the carbohydrate are monosaccharides, such as glucose and fructose; disaccharides, such as maltose, sucrose, and oligosaccharides; polysaccharides such as dextrin; typical sugars such as cyclodextrin; sugar alcohols, such as, xylitol, sorbitol, and erythritol. Examples of the flavoring agent may be natural flavoring agents (thaumatin, and stevia extract (e.g., rebaudioside A, glycyrrhizin, etc.)) and synthetic flavoring agents (saccharin, aspartame, etc.) Also, the food composition of the present invention may contain various nutrients, vitamins, minerals (electrolytes), dietary constituents, flavoring agents, such as synthetic flavoring agents and natural flavoring agents, a coloring agent, an extender (cheese, chocolate, etc.), pectic acid and its salt, alginic acid and its salt, organic acids, a protective colloid thickener, a PH adjuster, a stabilizer, a preservative, glycerin, alcohol, a carbonating agent used for a carbonated drink, and the like. For example, when the food composition of the present invention is prepared as a drink, the drink may further contain citric acid, liquid fructose, sugar, glucose, acetic acid, malic acid, fruit juice, various kinds of plant extracts, and the like, in addition to the high-hardness mineral water, which is an active ingredient of the present invention.

Considering easy accessibility to food, the food composition of the present invention is very useful in the prevention and alleviation of constipation.

In accordance with another aspect of the present invention, there is provided a pharmaceutical composition for preventing or treating constipation, the pharmaceutical composition comprising: (i) a pharmaceutically effective amount of high-hardness mineral water prepared from deep ocean water or salty underground water; and (ii) a pharmaceutically acceptable carrier, wherein the high-hardness mineral has the following properties below:

(a) having a hardness value of 40-3000; and
(b) containing 65-700 mg/ℓ of magnesium, 20-280 mg/ℓ of calcium, and 15-300 mg/ℓ of potassium.

Since the mineral water contained in the pharmaceutical composition of the present invention is the same as the mineral water contained in the food composition of the present invention, the overlapping descriptions therebetween are omitted to avoid excessive complication of the specification due to repetitive descriptions thereof.

As used herein, the term "treatment" refers to all actions that alleviate or beneficially change the symptoms of constipation by administration of the composition.

The pharmaceutical composition of the present invention includes a pharmaceutically acceptable carrier. The pharmaceutically acceptable carrier contained in the pharmaceutical composition of the present invention is one conventionally used for the formulation, and examples thereof may include, but are not limited to, lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methyl cellulose, methyl hydroxybenzoate, propyl hydroxybenzoate, talc, magnesium stearate, and mineral oil. The pharmaceutical composition of the present invention may further contain, in addition to the above components, a lubricant, a wetting agent, a sweetening agent, a flavoring agent, an emulsifier, a suspending agent, a preservative, and the like. Suitable pharmaceutically acceptable carriers and agents are described in detail in *Remington's Pharmaceutical Sciences* (19th ed., 1995).

The pharmaceutical composition of the present invention may be administered orally or parenterally, and preferably, an oral administration method is employed. The composition of the present invention may be administered as an individual therapeutic agent or in combination with another therapeutic agent, and may be administered together with the conventional therapeutic agent sequentially or simultaneously. In addition, single or multiple dosing may be performed.

As used herein, the term "pharmaceutically effective amount" refers to an amount that is sufficient to treat diseases at a reasonable benefit-risk ratio, which is applicable to medical treatment. The level of the effective amount may be variously determined depending on factors including the formulation method, manner of administration, patient's age, weight, sex, and morbidity, food, time of administration, route of administration, excretion rate, and response sensitivity.

The pharmaceutical composition of the present invention may be formulated into a unit or multiple dosage form using a pharmaceutically acceptable carrier and/or excipient according to the method easily conducted by a person having ordinary skill in the art to which the present invention pertains. Here, the dosage form may be a solution in an oily or aqueous medium, a suspension, a syrup, or an emulsion, an extract, a powder, a granule, a tablet, or a capsule, and may further include a dispersant or a stabilizer.

In accordance with still another aspect of the present invention, there is provided a method for preventing, alleviating, or treating constipation, the method comprising a step of administering to a subject a composition comprising: (i) a pharmaceutically effective amount of high-hardness mineral water prepared from deep ocean water or salty underground water; and (ii) a pharmaceutically acceptable carrier, wherein the high-hardness mineral has the following properties below:

> (a) having a hardness value of 40-3000; and
> (b) containing 65-700 mg/ℓ of magnesium, 20-280 mg/ℓ of calcium, and 15-300 mg/ℓ of potassium.

Since the method of the present invention uses the foregoing composition, descriptions of overlapping contents between the two are omitted to avoid excessive complication of the specification due to repetitive descriptions thereof.

Advantageous Effects

Features and advantages of the present invention are summarized as follows:

> (i) The present invention provides a functional composition for preventing, alleviating, or treating constipation, the functional composition containing, as an active ingredient, high-hardness mineral water prepared from deep ocean water or salty underground water.
> (ii) Since the composition of the present invention contains large quantities of magnesium, calcium, and potassium, the ingestion of the mineral water improves fecal parameters, and thus, the composition of the present invention can prevent, alleviate, and treat constipation.
> (iii) The present invention is safe to the human body by containing, as an active ingredient, high-hardness mineral water prepared from deep ocean water or salty underground water, and thus can be useful for the treatment of constipation for a cancer patient.

Brief Description of the Drawings

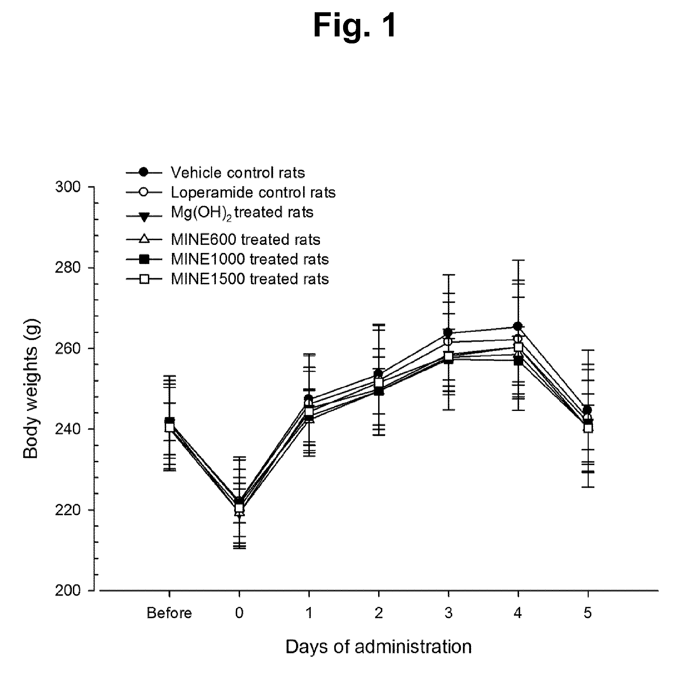

FIG. 1 shows the change in body weight in the loperamide-induced rat model.

Respective values are expressed as mean ± S.D. for nine rats; MINE600, MINE1000, and MINE1500 represent high-hardness mineral water prepared using bedrock water of the deep ocean; arrow represents fasting for one night; "Before" represents one day prior to initiation of administration; and "0" represents the data of initiation of administration.

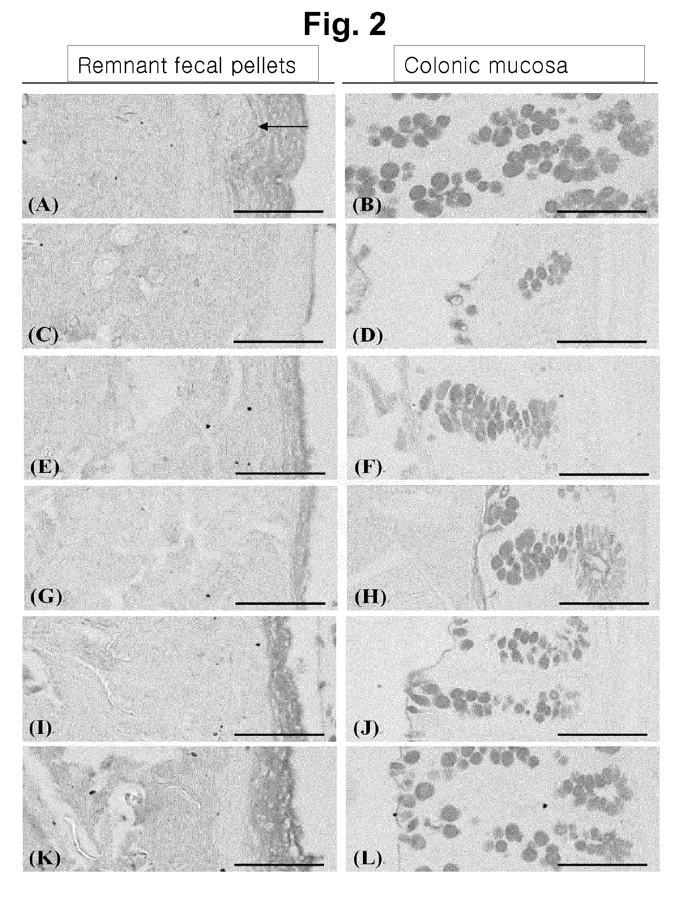

FIG. 2 shows representative histological profiles of the fecal pellet-containing colons in the loperamide-induced constipated rat model.

> A, B: Normal group
> C, D: Loperamide control;
> E, F: $Mg(OH)_2$ administered rat;
> G, H: MINE600 mineral water administered rat;
> I, J: MINE1000 mineral water administered rat;
> K, L: MINE1500 mineral water administered rat, and

The arrow represents the measured surface mucous thickness, and scale bar represents 100 μm.

Mode for Carrying Out the Invention

Hereinafter, the present invention will be described in detail with reference to examples. These examples are only for illustrating the present invention more specifically, and it will be apparent to those skilled in the art that the scope of the present invention is not limited by these examples.

**Examples**

**Materials and Methods**

*Animals*

6-week-old rats (SLC, Japan) were used for an experiment after acclimatization to new environments for 7 days. Five rats were raised per cage at 20-25°C and 45-50% humidity. The light-dark cycle was 12hr : 12hr, and water and food were freely available. The rats were classified into six groups (nine rats per group, a total of 54 rats) based on the body weight thereof. Specific groups are as follows:

<Group 1> Physiological saline and sterile distilled water administered control (30 m$\ell$/kg/day)
<Group 2> Loperamide 3 mg/kg + sterile distilled water 30 m$\ell$/kg/day administered group
<Group 3> Loperamide 3 mg/kg + Mg(OH)$_2$ 200 mg/kg/day administered group
<Group 4> Loperamide 3 mg/kg + MINE600 30 m$\ell$/kg/day administered group
<Group 5> Loperamide 3 mg/kg + MINE1000 30 m$\ell$/kg/day administered group
<Group 6> Loperamide 3 mg/kg + MINE1500 30 m$\ell$/kg/day administered group.

*Preparation of high-hardness mineral water*

<Separation of calcium salt crystals and salt from concentrated water of deep ocean water>

Deep ocean water is separated into concentrated water and desalted water through a reverse osmosis system. The obtained concentrated water was heated and concentrated to start the extraction of calcium salts. The calcium salts were extracted from a specific gravity of 1.11 (using a gravimeter). A 300-mesh net was used at the time of extraction. After that, the concentrated water, from which calcium salts had been removed, was further heated and concentrated to obtain a yellowish mineral concentrate in which elements of salt (NaCl) were removed and ion components (minerals) were concentrated. Specifically, the concentrated water, from which calcium salts were removed, was continuously heated to have a specific gravity of 1.32. Here, the mineral concentrate (liquid) and salt (solid) are present together in the heated concentrated water, and was separated into solid and liquid using a centrifuge or a dehydrator.

<Removal of calcium carbonate and elements of salt from calcium salt crystals>

The separated calcium salt crystals contain large quantities of elements of salt since it has been separated from the concentrated water obtained by performing a desalting treatment on the deep ocean water. Such elements of salt degrade the eating texture, and thus act as a factor to restrict the preparation of high-hardness mineral water. Hence, the present inventors conducted as follows in order to remove calcium carbonate and elements of salt from calcium salt crystals. First, the calcium salt crystals separated from the concentrated water were put in a container which contained 70°C hot water (having 10-fold volume of calcium salt crystals) and were equipped with a 300-mesh net (custom-made). The container was smoothly shaken so that the calcium salt crystals could mix well with the hot water. After that, the calcium salt crystals remaining on the 300-mesh net were collected, followed by water removal and drying in an oven, so that the calcium salt crystals were prepared in a state in which they can be stored for a long time. When the calcium salt crystals were purified as described above, the elements of salt put on the calcium salt crystals were dissolved in the hot water, and calcium carbonate passed through the net and were deposited on the bottom.
In order to investigate the difference between the calcium salt crystals separated from the concentrated water and the calcium salt crystals purified by the above method, the calcium salts for each case were dissolved in 1$\ell$ of distilled water based on a hardness of 500, and then the quantity of calcium contained in the distilled water was measured (0.86 g of calcium salt crystals were dissolved before and after purification, respectively, and moisture was maximally removed from the calcium salt crystals before purification). The measurement results are shown in table 1.

[Table 1]

|  | Before purification | After purification |
|---|---|---|
| Ca (mg/$\ell$) | 351.7 | 496 |

As shown in table 1, a larger quantity of calcium was detected in the distilled water where un-purified calcium salt crystals were dissolved than in the distilled water where calcium salt crystals having gone through the purification procedure were dissolved. Such a result represents an almost a similar value to $CaSO_4$, as a food additive, and indicates that the calcium salts separated from the deep ocean water were purified by the method of the present invention to obtain well-soluble high-purity calcium. Thus, according to the present invention, mineral water containing a larger quantity of calcium can be provided. In addition, such results indicate that Na, which has an unnecessary effect on the eating texture of the mineral water, can be excluded.

<Quality improvement of mineral concentrate>

The mineral concentrate, from which the elements of salt have been removed, was heated at 70-80°C after a vacuum was made, to have a specific gravity of 1.32, thereby further concentrating the mineral concentrate. After the concentration, impurities and anions were removed from the mineral concentrate to improve quality of the mineral concentrate. Specifically, 100$\ell$ of the mineral concentrate was passed through an activated carbon filter (10-inch, Saehan Filter). The mineral concentrate passed through the activated carbon filter once was sequentially passed through an activated carbon filter and a sterilizing filter (0.2 $\mu$m, 10-inch, Saehan Filter). Finally, as a result of observing the color of the mineral concentrate passed through the sterilizing filter, the yellowish mineral concentrate before passing through the filter was changed into a clear mineral concentrate after passing through the filter. Such a result indicates that silt was removed from the mineral concentrate by the quality improving procedure as described above.

In addition, in order to verify whether anions in the mineral concentrate were removed by the method, the quantities of $Cl^-$ and $SO_4^{2-}$ before and after passing through filter were measured. The measurement results are shown in table 2.

[Table 2]

|  | Before treatment | After treatment | reduction | |
|---|---|---|---|---|
| $Cl^-$ | 291,248 | 272,246 | 19,002 | 6.52% |
| $SO_4^{2-}$ | 115,830 | 113,425 | 2,405 | 2.08% |

As shown in table 2, it was verified that considerable quantities of chlorine ions and sulfate ions were removed by filter passage. Such results indicate that the anions in the concentrate can be effectively removed by filtering the mineral concentrate through a filter having a physical adsorbent → re-filtering the filtered concentrate through a filter having a physical adsorbent → filtering the re-filtered concentrate through a hollow fiber membrane filter. Since the anions are a factor to degrade the eating texture of the mineral water, the anions are removed by the method of the present invention, thereby improving the refreshing feeling of the mineral water, and further allowing the preparation of high-hardness water from deep ocean water.

<Preparation of high-hardness mineral water>

The obtained mineral concentrate, from which silt, $Cl^-$, and $SO_4^{2-}$ have been removed, and the obtained purified calcium salt crystals were mixed with desalted water to prepare mineral waters with hardness (mg/$\ell$) of 600 (MINE600), 1000 (MINE1000), and 1500 (MINE1500). The mineral contents of mineral water MINE600 were analyzed, and the results are shown in table 3.

[Table 3]

| Mineral content | mg/$\ell$ |
|---|---|
| Magnesium (Mg) | 101-140 |
| Calcium (Ca) | 32-49 |
| Potassium (K) | 29-54 |

*Administration of test material*

Magnesium hydroxide (Mg(OH)$_2$; MagMill™) was purchased from Samnam Pharm. In order to protect test materials from light and moisture, the test materials were stored in a refrigerator at 4°C. The mineral water was orally administered at 10 m$\ell$/kg for each time, at an interval of 6 hours (30 m$\ell$/kg/day), three times a day, for a total of six days. The administration for each time was conducted 1 hour after administration of loperamide 3 mg/kg. Magnesium hydroxide was mixed with distilled water, and the mixture was orally administered once a day for a total of six days, and the same amount of distilled water was orally administered at the time of second and third administrations. Distilled water, instead of mineral water, was administered to groups 1 and 2.

*Manufacture of constipation-induced animal model*

In order to induce constipation, loperamide hydrochloride (Sigma, MO, U.S.) 3 mg/kg was mixed with saline water 5 m$\ell$/kg, and the mixture was orally administered to animals 1 hour before administration of the test material, once a day, for a total of six days. Detailed methods are disclosed in Bustos et al. Acta Gastroenterol Latinoam, 1991;21:3-9 and Wintola et al. BMC Gastroenterol, 2010;10:95, which are incorporated by reference into the present specification.

*Measurement of body weight change*

Body weight was measured once per day 1 day before the test material administration, at the day of the initiation of administration, and 1, 2, 3, 4, and 5 days after the initiation of administration, to verify the body weight change according to administration of the test material. All the animals were fasted (excluding water) for 5 days after the initiation of administration for one overnight in order to decrease the body weight difference due to feed ingestion and measure the intestinal charcoal transit ratio. In addition, the body weight gain was calculated by equation 1.

[Equation 1]

Body weight gain (g) = body weight at 6$^{th}$ administration last day − body weight at the day of the initiation of administration.

*Fecal parameter verification*

The fecal pellets of each rat were collected 24 hours after the 4$^{th}$ administration day. The total number, wet weight, and water content of the fecal pellets were checked. The water content was calculated by equation 2 below:

[Equation 2]

Water content of fecal pellets (%) = ((wet weight of fecal pellets − dry weight of fecal pellets)/wet weight of fecal pellets) x 100

*Measurement on gastrointestinal charcoal transit ratio*

The gastrointestinal propulsion of the charcoal meal was checked by the method disclosed in Sagar et al. BMC Complement Altern Med, 2005; 5:18, with minor modification. Prior to the experiment, animals were fasted for 18 hours (water supplied). Ten minutes after the 6$^{th}$ administration (last) of the test material, animals of each group were fed with 1 m$\ell$ of charcoal meal (3% suspension of activated charcoal in 0.5% aqueous methylcellulose). Thirty minutes after charcoal meal ingestion, the animals of each group were sacrificed, and the total small intestine length (from pyloric sphincter to appendix) and the charcoal meal transit distance were measured. The gastrointestinal charcoal transit ratio was calculated by equation 3 below:

[Equation 3]

Charcoal transit ratio (%) = ((total small intestine length − charcoal meal transit distance)/total small intestine length) x 100

*Measurement on fecal pellets in the large intestine*

The total number and mean thickness (short axis) of remnant fecal pellets in the colonic lumen were measured when the gastrointestinal charcoal transit ratio was measured.

*Verification on histopathological change*

Histopathological assessment of the colonic mucosa and remnant fecal pellets in the colonic lumen was conducted by the method of Wu et al. J Ethnopharmacol, 2011; 134:406-13, with minor modification. In short, the distal colon fragment containing one fecal pellet was isolated using ligatures, removed, and immediately fixed with 10% formaldehyde (when the gastrointestinal charcoal transit ratio was measured). The fixed tissue segments were paraffin-embedded, and sliced into 3 $\mu$m-thickness cross sections. The sliced samples were stained with Alcian blue at pH 2.5. Five tissue segments per group were prepared, and histological profiles thereof were interpreted. The histopathologist was blinded to the group distributions at the time of analysis.

Histomorphometry: The mean thickness of the mucosal layers at the fecal surface ($\mu$m/fecal pellet), mucous-producing cell number (cells/mm$^2$) per unit area in the colonic mucosa, and the colonic mucosal thickness ($\mu$m/colon) were measured by using an automated image analyzer (DMI-300, DMI, Korea) under a light microscope.

*Statistical analysis*

Multiple comparison tests of the groups with different dosages were conducted. Variance homogeneity was examined using the Levene test (Levene A, Clin Otalary, 1981; 6:145-51). If the Levene test indicated no significant deviations from variance homogeneity, the obtained data were analyzed by one-way ANOVA, followed by the least significant differences (LSD) multiple comparisons test to determine which group pairs differed significantly. In cases where significant deviations from variance homogeneity were observed by the Levene test, the non-parametric comparison test and Kruskal-Wallis H test were conducted. When a significant difference was observed by the Kruskal-Wallis H test, the Mann-Whitney U (MW) test was used to determine the significance of specific pairwise comparisons. Statistical analyses were conducted using SPSS (Release 14K, SPSS Inc. USA; Ludbrook, Clin Exp Pharmacol Physiol, 1997; 24:294-6). In addition, the percentage change between the normal group and loperamide control was calculated to identify the severity of induced constipation, and then, in order to evaluate the efficacy of the test materials, the percent change compared with the loperamide control was calculated by equations 4 and 5.

[Equation 4]

Percent change compared with normal group (%) = (((normal group data)-(loperamide control data)/normal group data) x 100)

[Equation 5]

Percentage change (%) compared with loperamide control = (((loperamide control data − test material administered rat data)/(loperamide control data)) × 100)

Results

*Effects on body weight*

Significant changes in body weight and weight gain were not observed in constipation-induced rats with loperamide treatment (groups 2-6) compared with normal rats (group 1). As a result of comparison with the normal group and loperamide control (group 2), any test material that can induce significant changes in body weight and weight gain was not detected in the present experiment (table 1 and FIG. 1).

[Table 4]

Body weight change of loperamide-induced constipated rats

| Groups | Body weights at Initiation of administration [A] | Fifth administration | Last (6th) administration[B] | Body weight gains [B-A] |
|---|---|---|---|---|
| Controls | | | | |
| Vehicle | 222.11±10.25 | 265.33±16.55 | 244.56±14.99 | 22.44±6.13 |
| Loperamide | 222.00±11.10 | 262.22±14.66 | 242.67±13.40 | 20.67±5.68 |
| Reference | | | | |
| Mg(OH)$_2$ | 219.22±8.79 | 260.33±12.38 | 241.67±10.36 | 22.44±4.19 |
| MINE600 | 219.33±5.87 | 258.36±6.80 | 240.33±8.47 | 21.00±5.32 |
| MINE1000 | 221.67±4.92 | 257.00±6.00 | 240.44±5.53 | 18.78±5.54 |
| MINE1500 | 220.56±9.48 | 260.33±15.60 | 240.22±14.58 | 19.67±6.40 |

Values are expressed mean ± S.D. of nine rats, g

As shown in table 4 and FIG. 1, the change in weight gain during the administration period changed by -7.92% in the loperamide control, and by 8.60, 1.61, -9.14, and -4.84% in groups 3-6, respectively, as compared with the loperamide control.

*Effects on fecal parameters*

As shown in table 5, significant (p<0.01) decreases in the number and water content of fecal pallets were observed in the loperamide control compared with the normal group, but significant (p<0.01 or p<0.05) increases in the number and water content of fecal pellets were observed in group 3 (Mg(OH)$_2$ administered group) and groups 5 and 6 (mineral water-administered group) compared with the loperamide control. In addition, a significant (p<0.05) increase in the number of fecal pallets was observed and a remarkable increase in the water content of fecal pellets was also observed in group 3 compared with the normal group. Specifically, the number of fecal pellets for 24 hours, 4 days after administration, changed by -27.45% in the loperamide control compared with the normal group, and by 17.94, 17.20, 21.38, and 24.32% in group 3 (Mg(OH)$_2$ administered group) and groups 4 to 6 (mineral water-administered groups) compared with the loperamide control, respectively.

In addition, the water content in fecal pellets for 24 hours, 4 days after administration, changed by -17.47% in the loperamide control compared with the normal group, and by 18.12, 12.01, 22.68, and 24.01% in group 3 (Mg(OH)$_2$ administered group) and groups 4 to 6 (mineral water-administered groups) compared with the loperamide control, respectively.

[Table 5]

Fecal parameters in loperamide-induced constipated rats

| Groups | Fecal Parameters collected at 4th administration day (24 hrs) | | | |
|---|---|---|---|---|
| | Numbers of fecal pellets | Fecal wet-weights (g/24hr/rat) | Fecal dry-weights (g/24hr/rat) | Water contents (%) |
| Controls | | | | |
| Vehicle | 62.33±8.37 | 11.59±1.07 | 5.19±0.83 | 54.88±8.37 |
| Loperamide | 45.22±4.97[d] | 7.65±1.25[d] | 4.17±0.63[a] | 45.29±4.06[a] |
| Reference | | | | |
| Mg(OH)$_2$ | 52.33±4.21[ef] | 9.18±0.88[dg] | 4.21±0.55[a] | 53.50±9.99[c] |
| MINE600 | 53.00±6.50[eg] | 8.99±0.90[dg] | 4.42±0.53[b] | 50.73±4.28 |
| MINE1000 | 54.89±3.92[ef] | 9.89±1.07[df] | 4.39±0.61[b] | 55.56±4.83[c] |

(continued)

Fecal parameters in loperamide-induced constipated rats

| Groups | Fecal Parameters collected at 4th administration day (24 hrs) | | | |
| --- | --- | --- | --- | --- |
| | Numbers of fecal pellets | Fecal wet-weights (g/24hr/rat) | Fecal dry-weights (g/24hr/rat) | Water contents (%) |
| MINE1500 | 56.22+9.27[f] | 10.64±2.21[ac] | 4.60±0.68 | 56.16±4.39[c] |

Values are expressed mean ± S.D. of nine rats
[a]$p < 0.01$ and [b]$p < 0.05$ as compared with vehicle control by LSD test
[c]$p < 0.01$ as compared with loperamide control by LSD test
[d]$p < 0.01$ and [e]$p < 0.05$ as compared with vehicle control by MW test
[f]$p < 0.01$ and [g]$p < 0.05$ as compared with loperamide control by MW test

*Effects on Remnant fecal pellets in the colonic lumen*

As shown in table 6, significant ($p < 0.01$) increases in the number and mean diameter of remnant fecal pellets in the colon were observed in the loperamide control compared with the normal group, but significant ($p < 0.01$) decreases in the number and mean diameter of remnant fecal pellets in the colon 18h after fasting were observed in groups 3 to 6 compared with the loperamide control. Specifically, the number of remnant fecal pellets in the colon changed by 118.75% in the loperamide control compared with the normal group, and by -51.43, -37.14, - 62.86, and -62.86% in groups 3 to 6 compared with the loperamide control, respectively.
In addition, the mean diameter of remnant fecal pellets in the colon changed by 57.96% in the loperamide control compared with the normal group, and by -18.76, -15.71, -25.09, and -27.53% in groups 3 to 6 compared with the loperamide control, respectively (table 6).

[Table 6]

Remnant fecal pellets in loperamide-induced constipated rats

| Groups | Remnant fecal pellets in the colon | |
| --- | --- | --- |
| | Numbers | Mean thicknesses ($\mu$m) |
| Controls | | |
| Vehicle | 1.78±0.83 | 4.10±0.66 |
| Loperamide | 3.89±1.05[a] | 6.48±0.73[a] |
| Reference | | |
| Mg(OH)$_2$ | 1.89±0.93[c] | 5.26±0.38[c] |
| MINE600 | 2.44±1.01[c] | 5.46+0.65[c] |
| MINE1000 | 1.44±1.01[c] | 4.85±0.64[bc] |
| MINE1500 | 1.44±0.73[c] | 4.70±0.55[bc] |

Values are expressed mean ± S.D. of nine rats
[a]$p < 0.01$ and [b]$p < 0.05$ as compared with vehicle control by LSD test
[c]$p < 0.01$ as compared with loperamide control by LSD test

*Effects on Intestinal Charcoal Transit*

As shown in table 7, a significant ($p < 0.01$) decrease in the intestinal charcoal transit ratio was observed in the loperamide control compared with the normal group, but significant ($p < 0.01$ or $p < 0.05$) increases in the intestinal charcoal transit ratio for 6 days of administration of test materials were observed in group 3 (Mg(OH)$_2$ administered group) and groups 4 to 6 (mineral water-administered group) compared with the loperamide control, respectively. Specifically, the intestinal charcoal transit ratio changed by -20.05% in the loperamide control compared with the normal group, and by 9.10, 9.63, 21.42, and 22.03% in groups 3 to 6 compared with the loperamide control, respectively (table 7).

[Table 7]

Gastrointestinal charcoal transit ratio in loperamide-induced constipated rats

| Groups | Gastrointestinal motilities (during 30min) | | |
|---|---|---|---|
| | Total small intestine length (cm) | Length of charcoal meal transferred (cm) | Gastrointestinal charcoal transit ratio (%) |
| Controls | | | |
| Vehicle | 122.30±5.21 | 93.44±5.43 | 76.55±5.86 |
| Loperamide | 121.40±2.90 | 74.21±6.41[a] | 61.20±5.98[a] |
| Reference | | | |
| Mg(OH)$_2$ | 124.81+6.10 | 83.333±6.06[a] | 66.76±3.47[ac] |
| MINE600 | 121.97±3.81 | 81.80±6.25[ac] | 67.09±4.96[ac] |
| MINE1000 | 123.74+3.74 | 91.90±7,33[b] | 74.30±5.99[b] |
| MINE1500 | 123.77±5.78 | 92.39±6.54[b] | 74.68±4.60[b] |

Values are expressed mean±S.D. of nine rats
[a]p<0.01 as compared with vehicle control by LSD test
[b]p<0.01 and [c]p =0.05 as compared with loperamide control by LSD test

*Effects on Histopathological change*

As shown in table 8 and FIG. 2, significant (p<0.01) decreases in the fecal pellet surface mucosal thickness in the colon, mucous-producing cell number per unit area in the colonic mucosa, and the colon mucosa thickness were observed in the loperamide control compared with the normal group, but significant (p<0.01) increases in the fecal pellet surface mucosal thickness in the colon, mucous-producing cell number per unit area in the colonic mucosa, the colon mucosa thickness, for 6 days of administration of test materials, were observed in group 3 (Mg(OH)$_2$ administered group) and groups 4 to 6 (mineral water-administered groups) compared with the loperamide control, respectively. Specifically, the fecal pellet surface mucosal thickness in the colon changed by -89.93% in the loperamide control compared with the normal group, and by 316.22, 308.84, 424.83, and 600.59% in groups 3 to 6 compared with the loperamide control, respectively. In addition, the mucous-producing cell number per unit area in the colonic mucosa changed by -72.40% in the loperamide control compared with the normal group, and by 75.17, 45.56, 96.71, and 141.90% in groups 3 to 6 compared with the loperamide control, respectively.

Furthermore, the colon mucosa thickness changed by -70.97% in the loperamide control compared with the normal group, and by 57.27, 37.74, 71.13, and 122.43% in groups 3 to 6 compared with the loperamide control, respectively (table 8 and FIG. 2).

[Table 8]

Histomorphometric results of the colon and remnant fecal pellets in loperamide-induced constipated rats

| Groups | Histomorphometry (at sacrifice) | | |
|---|---|---|---|
| | Fecal pellet surface mucous thicknesses ($\mu$m) | Mucous producing cell numbers (cells/mm$^2$) | Colon mucosa, thicknesses ($\mu$m) |
| Controls | | | |
| Vehicle | 58.01±11.04 | 648.56+85.75 | 359,61±66.13 |
| Loperamide | 5.84±2.53[a] | 179.00±46.31[c] | 104.38±10.79[c] |
| Reference | | | |
| Mg(OH)$_2$ | 24.33±7.82[ab] | 313.56±42.27[cd] | 164.16±22.31[cd] |
| MINE600 | 23.89±5.36[ab] | 260.56±33.48[cd] | 143.78±13.77[cd] |
| MINE1000 | 30.67±5.41[ab] | 352.11±40.77[cd] | 179.67±24.72[cd] |

(continued)

Histomorphometric results of the colon and remnant fecal pellets in loperamide-induced constipated rats

| Groups | Histomorphometry (at sacrifice) | | |
|---|---|---|---|
| | Fecal pellet surface mucous thicknesses ($\mu$m) | Mucous producing cell numbers (cells/mm$^2$) | Colon mucosa, thicknesses ($\mu$m) |
| MINE1500 | 40,95$\pm$10.21[ab] | 433.00$\pm$46.01[cd] | 232.18$\pm$64.41[cd] |

Values are expressed mean $\pm$ S.D. of nine rats
[a]$p < 0.01$ as compared with vehicle control by LSD test
[b]$p < 0.01$ as compared with loperamide control by LSD test
[c]$p < 0.01$ as compared with vehicle control by MW test
[d]$P < 0.01$ as compared with Loperamide control by MW test

Although the present invention has been described in detail with reference to the specific features, it will be apparent to those skilled in the art that this description is only for a preferred embodiment and does not limit the scope of the present invention. Thus, the substantial scope of the present invention will be defined by the appended claims and equivalents thereof.

**Claims**

1.  A composition for preventing or alleviating constipation, containing, as an active ingredient, high-hardness mineral water prepared from deep ocean water or salty underground water, wherein the high-hardness mineral water has the following properties:

    (a) having a hardness value of 40-3000; and
    (b) containing 65-700 mg/$\ell$ of magnesium, 20-280 mg/$\ell$ of calcium, and 15-300 mg/$\ell$ of potassium.

2.  The composition of claim 1, wherein the high-hardness mineral water, (a) has a hardness value of 500-2000; and contains 80-480 mg/$\ell$ of magnesium, 25-170 mg/$\ell$ of calcium, and 20-200 mg/$\ell$ of potassium.

3.  The composition of claim 1, wherein the high-hardness mineral water exhibits the following activities:

    (a) an increase in the weight of fecal pellets;
    (b) an increase in the water content of fecal pellets;
    (c) a decrease in the number of remnant fecal pellets in the colon;
    (d) an increase in the intestinal fecal pellet transit ratio; and
    (e) increases in the fecal pellet surface mucous thickness, colon mucosa thickness, and mucous-producing cell number per unit area in colon mucosa.

4.  The composition of claim 1, wherein the composition prevents or alleviates constipation of a cancer patient.

5.  The composition of claim 1, wherein the high-hardness mineral water is prepared to have a hardness of 400-3000 by mixing desalted water, which is obtained by performing a desalting treatment on deep ocean water or salty underground water, with (i) a mineral concentrate obtained by separating calcium salt crystals and salt from concentrated water obtained by performing a desalting treatment on deep ocean water or salty underground water; and (ii) calcium salts obtained by removing, from the calcium salt crystals separated from the concentrated water, salt put on the calcium salt crystals and calcium carbonate, and wherein the mineral concentrate is obtained by filtering the mineral concentrate through a filter having a physical adsorbent, re-filtering the filtered mineral concentrate through a filter having a physical adsorbent, and then filtering the re-filtered mineral concentrate through a hollow fiber membrane filter having a plurality of pores.

6.  The composition of claim 5, wherein the separation of the calcium salt crystals and salt from the concentrated water is performed by heating and concentrating the concentrated water to have a specific gravity of 1.1-1.23, thereby extracting and separating the calcium salt crystals, and then re-heating and re-concentrating the concentrated water to have a specific gravity of 1.24-1.32, thereby extracting and separating the salt.

7. The composition of claim 5, wherein the step of filtering the mineral concentrate through the filter having a physical adsorbent and re-filtering the filtered mineral concentrate through the filter having a physical adsorbent is performed 1-5 times.

8. The composition of claim 5, wherein the removal of the salt and the calcium carbonate from the calcium salt crystals separated from the concentrated water is performed by putting the calcium salt crystals separated from the concentrated water in a container equipped with a 300- to 350-mesh net and containing hot water, and separating calcium salt crystals that do not pass through the net, and here, the salt put on the calcium salt crystals separated from the concentrated water is dissolved in the hot water, and the calcium carbonate passes through the net.

9. A pharmaceutical composition for preventing or treating constipation, the pharmaceutical composition comprising: (i) a pharmaceutically effective amount of high-hardness mineral water prepared from deep ocean water or salty underground water; and (ii) a pharmaceutically acceptable carrier, wherein the high-hardness mineral has the following properties below:

   (a) having a hardness value of 40-3000; and
   (b) containing 65-700 mg/ℓ of magnesium, 20-280 mg/ℓ of calcium, and 15-300 mg/ℓ of potassium.

10. A method for preventing, alleviating, or treating constipation, the method comprising a step of administering to a subject a composition comprising:

    (i) a pharmaceutically effective amount of high-hardness mineral water prepared from deep ocean water or salty underground water; and (ii) a pharmaceutically acceptable carrier, wherein the high-hardness mineral has the following properties below:

    (a) having a hardness value of 40-3000; and
    (b) containing 65-700 mg/ℓ of magnesium, 20-280 mg/ℓ of calcium, and 15-300 mg/ℓ of potassium.

11. The method of claim 10, wherein the high-hardness mineral water (a) has a hardness value of 500-2000; and contains 80-480 mg/ℓ of magnesium, 25-170 mg/ℓ of calcium, and 20-200 mg/ℓ of potassium.

12. The method of claim 10, wherein the high-hardness mineral water exhibits the following activities:

    (a) an increase in the weight of fecal pellets;
    (b) an increase in the water content of fecal pellets;
    (c) a decrease in the number of remnant fecal pellets in the colon;
    (d) an increase in the intestinal fecal pellet transit ratio; and
    (e) increases in the fecal pellet surface mucous thickness, colon mucosa thickness, and mucous-producing cell number per unit area in colon mucosa.

13. The method of claim 10, wherein the composition prevents or alleviates constipation of a cancer patient.

14. The method of claim 10, wherein the high-hardness mineral water is prepared to have a hardness of 400-3000 by mixing desalted water, which is obtained by performing a desalting treatment on deep ocean water or salty underground water, with (i) a mineral concentrate obtained by separating calcium salt crystals and salt from concentrated water obtained by performing a desalting treatment on deep ocean water or salty underground water; and (ii) calcium salts obtained by removing, from the calcium salt crystals separated from the concentrated water, salt put on the calcium salt crystals and calcium carbonate, and wherein the mineral concentrate is obtained by filtering the mineral concentrate through a filter having a physical adsorbent, re-filtering the filtered mineral concentrate through a filter having a physical adsorbent, and then filtering the re-filtered mineral concentrate through a hollow fiber membrane filter having a plurality of pores.

15. The method of claim 14, wherein the separation of the calcium salt crystals and salt from the concentrated water is performed by heating and concentrating the concentrated water to have a specific gravity of 1.1-1.23, thereby extracting and separating the calcium salt crystals, and then re-heating and re-concentrating the concentrated water to have a specific gravity of 1.24-1.32, thereby extracting and separating the salt.

16. The method of claim 14, wherein the step of filtering the mineral concentrate through the filter having a physical adsorbent and re-filtering the filtered mineral concentrate through the filter having a physical adsorbent is performed 1-5 times.

17. The method of claim 14, wherein the removal of the salt and the calcium carbonate from the calcium salt crystals separated from the concentrated water is performed by putting the calcium salt crystals separated from the concentrated water in a container equipped with a 300- to 350-mesh net and containing hot water, and separating calcium salt crystals that do not pass through the net, and here, the salt put on the calcium salt crystals separated from the concentrated water is dissolved in the hot water, and the calcium carbonate passes through the net.

# Fig. 1

# Fig. 2

| Remnant fecal pellets | Colonic mucosa |
|---|---|

## INTERNATIONAL SEARCH REPORT

International application No.

**PCT/KR2013/009388**

### A. CLASSIFICATION OF SUBJECT MATTER

***A23L 1/304(2006.01)i, C02F 1/28(2006.01)i, C02F 1/44(2006.01)i***

According to International Patent Classification (IPC) or to both national classification and IPC

### B. FIELDS SEARCHED

Minimum documentation searched (classification system followed by classification symbols)

A23L 1/304; A23L 2/74; A23L 1/30; A23L 2/38; A23L 2/70; C01B 5/00; A61K 7/00; A61K 31/20; A61K 8/21; A61P 1/02; C02F 1/28; C02F 1/44

Documentation searched other than minimum documentation to the extent that such documents are included in the fields searched
Korean Utility models and applications for Utility models: IPC as above
Japanese Utility models and applications for Utility models: IPC as above

Electronic data base consulted during the international search (name of data base and, where practicable, search terms used)

eKOMPASS (KIPO internal) & Keywords: deep ocean water, saline groundwater, constipation, mineral water, magnesium, calcium, potassium, hardness value

### C. DOCUMENTS CONSIDERED TO BE RELEVANT

| Category* | Citation of document, with indication, where appropriate, of the relevant passages | Relevant to claim No. |
|---|---|---|
| A | KR 10-2005-0037049 A (LEE, Bong Sang) 21 April 2005<br>See abstract; claims 1-2; table 1. | 1-9 |
| A | JP 2002-262829 A (TOYAMA CHEMICAL CO., LTD.) 17 September 2002<br>See abstract; claims 1-5. | 1-9 |
| A | KR 10-2009-0119630 A (DONGGUK UNIVERSITY GYEONGJU CAMPUS INDUSTRY-ACADEMY COOPERATION FOUNDATION) 19 November 2009<br>See abstract; claims 1-3 and 6. | 1-9 |
| A | KR 10-0944538 B1 (OCIAD CO., LTD.) 03 March 2010<br>See abstract; claims 1-5. | 1-9 |
| A | US 6649176 B1 (SHAPIRO, Stanley S. et al.) 18 November 2003<br>See abstract; claims 1 and 3. | 1-9 |
| A | US 2009-0185986 A1 (CIESLIK, Tadeusz et al.) 23 July 2009<br>See abstract; claim 1. | 1-9 |

☐ Further documents are listed in the continuation of Box C.     ☒ See patent family annex.

| | | |
|---|---|---|
| * | Special categories of cited documents: | |
| "A" | document defining the general state of the art which is not considered to be of particular relevance | "T" later document published after the international filing date or priority date and not in conflict with the application but cited to understand the principle or theory underlying the invention |
| "E" | earlier application or patent but published on or after the international filing date | "X" document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search | Date of mailing of the international search report |
|---|---|
| 27 FEBRUARY 2014 (27.02.2014) | **27 FEBRUARY 2014 (27.02.2014)** |

| Name and mailing address of the ISA/KR | Authorized officer |
|---|---|
| Korean Intellectual Property Office<br>Government Complex-Daejeon, 189 Seonsa-ro, Daejeon 302-701, Republic of Korea | |
| Facsimile No. 82-42-472-7140 | Telephone No. |

Form PCT/ISA/210 (second sheet) (July 2009)

INTERNATIONAL SEARCH REPORT

International application No.

**PCT/KR2013/009388**

**Box No. II    Observations where certain claims were found unsearchable (Continuation of item 2 of first sheet)**

This international search report has not been established in respect of certain claims under Article 17(2)(a) for the following reasons:

1. ☒ Claims Nos.: **10-17**
   because they relate to subject matter not required to be searched by this Authority, namely:

   Claims 10-17 pertain to a method for treatment of the human by therapy, and thus pertain to subject matter on which the International Searching Authority is not required to carry out an international search under the provisions of PCT Article 17 (2)(a)(i) and PCT Rule 39.1(iv).

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III    Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**    ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (July 2009)

INTERNATIONAL SEARCH REPORT
Information on patent family members

International application No.

**PCT/KR2013/009388**

| Patent document cited in search report | Publication date | Patent family member | Publication date |
|---|---|---|---|
| KR 10-2005-0037049 A | 21/04/2005 | NONE | |
| JP 2002-262829 A | 17/09/2002 | NONE | |
| KR 10-2009-0119630 A | 19/11/2009 | KR 10-1003856 B1 | 23/12/2010 |
| KR 10-0944538 B1 | 03/03/2010 | NONE | |
| US 6649176 B1 | 18/11/2003 | AT 226063 T | 15/11/2002 |
| | | DE 60000616 D1 | 21/11/2002 |
| | | DE 60000616 T2 | 26/06/2003 |
| | | DE 60000616 T3 | 03/05/2007 |
| | | DK 1166762 T3 | 17/02/2003 |
| | | EP 1166762 A1 | 02/01/2002 |
| | | EP 1166762 B1 | 16/10/2002 |
| | | EP 1166762 B2 | 23/08/2006 |
| | | ES 2187428 T3 | 16/06/2003 |
| | | PT 1166762 E | 31/03/2003 |
| US 2009-0185986 A1 | 23/07/2009 | AT 503394 T | 15/04/2011 |
| | | DE 602008005833 D1 | 12/05/2011 |
| | | EP 2080438 A1 | 22/07/2009 |
| | | EP 2080438 B1 | 30/03/2011 |
| | | ES 2363569 T3 | 09/08/2011 |
| | | SI 2080438 T1 | 30/09/2011 |

Form PCT/ISA/210 (patent family annex) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020120118453
- KR 1007206950
- KR 1020080012457
- KR 100886598

**Non-patent literature cited in the description**

- **BUSTOS et al.** *Acta Gastroenterol Latinoam,* 1991, vol. 21, 3-9
- **WINTOLA et al.** *BMC Gastroenterol,* 2010, vol. 10, 95
- **SAGAR et al.** *BMC Complement Altern Med,* 2005, vol. 5, 18
- **WU et al.** *J Ethnopharmacol,* 2011, vol. 134, 406-13
- **LEVENE A.** *Clin Otalary,* 1981, vol. 6, 145-51
- **LUDBROOK.** *Clin Exp Pharmacol Physiol,* 1997, vol. 24, 294-6